# FASCICULE DE BREVET EUROPEEN

(11) **EP 3 582 699 B1**
(45) Date de publication et mention de la délivrance du brevet: **02.12.2020**
(21) Numéro de dépôt: 18704259.3
(22) Date de dépôt: 16.02.2018
(51) Int. Cl.: A61B 17/132

(54) **GARROT À MANCHON ÉLASTIQUE**
ABBINDER MIT ELASTISCHER MANSCHETTE
TOURNIQUET WITH ELASTIC SLEEVE

(30) Priorité: 17.02.2017 FR 1751317
(43) Date de publication de la demande: 25.12.2019
(73) Titulaire: Sorbonne Université, 75006 Paris (FR); Centre National de la Recherche Scientifique (CNRS), 75016 Paris (FR); Institut National de la Santé et de la Recherche Médicale (INSERM), 75013 Paris (FR); Filix, 10150 Creney (FR)
(72) Inventeur: CHENEGROS, Guillaume, 78190 Trappes (FR); LIBERT, Nicolas, 75005 Paris (FR); CARRAS, Stéphane, 14110 Conde Sur Noireau (FR)
(74) Mandataire: Regimbeau
(86) Numéro de dépôt international: PCT/EP2018/053869
(87) Numéro de publication internationale: WO 2018/149958

(56) Documents cités:
- WO-A1-03/049623
- WO-A1-2015/001542
- FR-A- 858 665
- US-A1- 2003 065 357
- US-A1- 2010 100 025

## Description

### DOMAINE DE L'INVENTION

La présente invention concerne un garrot.

Un garrot est un dispositif destiné à interrompre la circulation du sang dans un membre - membre supérieur (bras, avant-bras, main) ou membre inférieur (cuisse, jambe, pied) - en comprimant des vaisseaux sanguins du membre par un élément positionné autour du membre.

### ETAT DE LA TECHNIQUE

On connaît de l'état de la technique un garrot dit « à tourniquet », comprenant une sangle de serrage et une barre de préhension. La sangle entoure un membre à garrotter, et la barre de préhension est reliée à la sangle de telle sorte qu'une mise en rotation de la barre de serrage sur elle-même a pour effet de serrer la sangle contre le membre, ce qui a pour effet de comprimer les vaisseaux sanguins.

Toutefois, un garrot à tourniquet a comme inconvénient majeur de ne pas pouvoir être facilement utilisé d'une seule main.

En effet, un garrot à tourniquet requiert une certaine force pour serrer suffisamment la sangle de façon à stopper une hémorragie. Or, une personne blessée devant ne peut pas toujours développer une telle force.

De plus, la sangle peut être serrée de façon excessive autour du bras lorsque la barre de préhension est tournée sur elle-même sur un nombre de tours trop élevé, et qu'il n'est pas facile de détecter un tel serrage excessif. Or, un serrage excessif est susceptible de créer des lésions tissulaires dans le membre garrotté.

Il existe par ailleurs des garrots pneumatiques comprenant un élément gonflable. La compression du membre à garrotter est exercée lorsque l'élément gonflable, agencé autour du membre, est gonflé. Toutefois, ces garrots ont pour inconvénient d'être sensibles aux variations d'altitude : les compressions exercées par un garrot pneumatique sur un même membre à deux altitudes différentes ne sont donc pas les mêmes.

Ces garrots pneumatiques sont par ailleurs complexes d'utilisation. Leur utilisation est même souvent réservée à un personnel qualifié. Il en résulte qu'une personne non qualifiée ne peut pas aisément se poser un tel garrot pneumatique.

WO2015/001542 divulgue un garrot selon le préambule de la revendication 1.

### EXPOSE DE L'INVENTION

Un but de l'invention est de proposer un garrot insensible aux variations d'altitude qu'une personne blessée peut s'auto-appliquer facilement et très rapidement.

Il est dès lors proposé un garrot comprenant :
- un support propre à entourer une première partie d'un membre,
- un manchon élastique propre à être maintenu dans une position étirée autour du support, le support empêchant une rétraction du manchon élastique,
- un élément de préhension mobile par rapport au support,
- un élément d'entraînement propre à entraîner le manchon élastique, lorsque l'élément de préhension est déplacé manuellement par rapport au support, depuis sa position étirée autour du support vers une position de garrottage dans laquelle le manchon élastique entoure une deuxième partie du membre, adjacente à la première partie du membre mais non entourée par le support, de sorte que le manchon élastique puisse se rétracter par retour élastique, et ainsi comprimer la deuxième partie du membre.

Lorsque le manchon élastique s'étend autour du support, le support empêche le manchon élastique de se rétracter. Il est donc possible de pré-positionner le garrot sur un membre d'une personne à titre préventif, sans que le manchon élastique ne comprime ce membre, et en vue d'une utilisation future.

Une fois que le support a été positionné autour de la première partie du membre d'un utilisateur, et si d'aventure cet utilisateur vient à être blessé de telle sorte qu'il faille garrotter la deuxième partie de ce membre, il lui suffit alors de saisir l'élément de préhension et de le déplacer par rapport au support, pour que le manchon élastique soit chassé, au moyen de l'élément d'entraînement, hors du support qui le maintenait dans un état étiré jusqu'à ce qu'il atteigne la deuxième partie du membre à garrotter. Comme la deuxième partie du membre n'est pas entourée par le support, le manchon élastique, jusqu'ici dans un état étiré, est libre de se rétracter pour comprimer les vaisseaux sanguins de la deuxième partie du membre.

Une fois pré-positionné sur le membre, ce garrot est ainsi très simple à déclencher, notamment par et sur un porteur du garrot.

Par ailleurs, comme la compression des vaisseaux sanguins est assurée par le manchon élastique, le garrot proposé n'est pas sensible aux variations d'altitude comme c'est le cas pour les garrots existants de type pneumatique.

L'élément d'entraînement comprend une première portion et être propre à adopter:
- une configuration rabattue dans laquelle la première portion s'étend entre le support et l'ensemble du manchon élastique maintenu dans la position étirée autour du support,
- une configuration déployée dans laquelle le manchon élastique, dans la position de garrottage, s'étend entre la première portion et la deuxième partie du membre lorsque le support entoure la première partie du membre.

Ainsi, l'élément de préhension est agencé par rapport à la première portion pour qu'un déplacement de l'élément de préhension par rapport au support fasse basculer la première portion de l'élément d'entraînement depuis la configuration rabattue vers la configuration déployée, de sorte à déplacer, par effet de levier, le manchon élastique depuis la position étirée vers la position de garrottage.

La première portion peut être propre à :
- dans la configuration rabattue, entourer le support de sorte que le manchon élastique maintenu dans la position étirée s'étende autour de la première portion,
- dans la configuration déployée, entourer le manchon élastique.

La première portion peut en outre être propre à prendre une forme annulaire s'étendant autour d'un axe dans la configuration déployée, et est réalisé dans un matériau non-extensible parallèlement à l'axe.

L'élément d'entraînement peut comprendre une deuxième portion prolongeant la première portion. Dans ce cas, dans la configuration rabattue, la deuxième portion est rabattue sur la première portion de sorte à former, entre la première et la deuxième portion de l'élément d'entraînement, une cavité pour loger le manchon élastique maintenu dans la position étirée.

Le garrot peut comprendre des moyens de verrouillage de la deuxième portion de l'élément d'entraînement dans la configuration rabattue sur la première portion de l'élément d'entraînement.

Les moyens de verrouillage peuvent comprendre :
- au moins un orifice formé dans la première portion,
- au moins un orifice formé dans la deuxième portion,
- un cordon propre à être passé à travers les deux orifices, le cordon présentant une portion d'extrémité libre susceptible d'être accessible depuis l'extérieur du garrot et d'être tirée manuellement pour retirer le cordon hors des orifices, de sorte à déverrouiller les deux portions l'une par rapport à l'autre.

Les orifices peuvent être disposés l'un par rapport à l'autre pour qu'une portion du cordon s'étendant depuis l'un des orifices jusqu'à l'autre orifice forme une butée empêchant le manchon élastique logé dans la cavité de sortir de la cavité.

L'élément d'entraînement peut comprendre une troisième portion qui prolonge la deuxième portion et qui est par ailleurs fixée à l'élément de préhension, la deuxième portion s'étendant alors entre le manchon élastique et la troisième portion dans la configuration rabattue.

Le déplacement de l'élément de préhension suscitant l'entraînement, par l'élément d'entraînement, du manchon élastique depuis la position étirée autour du manchon élastique vers la position de garrottage peut être un déplacement causé par une traction manuelle exercée sur l'élément préhension le long du membre.

L'élément de préhension peut comprendre un élément de fixation propre à fixer l'élément de préhension de manière amovible à un vêtement pendant que le manchon élastique est maintenu autour du support dans sa position étirée, et l'élément de préhension être fixé à l'élément d'entraînement pour empêcher, lorsque l'élément de préhension est fixé au vêtement, l'entraînement, par l'élément d'entraînement, du manchon élastique depuis sa position étirée autour du support vers sa position de garrottage.

L'élément de fixation est par exemple propre à former, avec un autre élément de fixation, une liaison velours-crochet.

L'élément de fixation peut être propre à fixer l'élément de préhension sur une portion de vêtement entourant une troisième partie du membre lorsque le support entoure la première partie du membre, les deuxièmes et troisième parties du membre étant situées de part et d'autre de la première partie du membre.

L'élément de préhension peut comprendre un rabat mobile par rapport à l'élément d'entraînement entre :
- une configuration dépliée dans laquelle l'élément de fixation est en regard du vêtement, de sorte à pouvoir être fixé au vêtement,
- une configuration repliée sur l'élément d'entraînement en direction de la position de garrottage.

Le support peut présenter une surface interne propre à être orientée vers la première partie du membre, et une surface externe opposée à la surface interne, et le garrot peut comprendre par ailleurs :
- un élément textile interne recouvrant la surface interne, et/ou
- un élément textile externe recouvrant la surface externe.

Les deux éléments textiles peuvent définir ensemble une poche pour contenir le support, la poche étant pourvue d'une ouverture par laquelle le support est susceptible d'être retiré de la poche.

Le garrot peut également comprendre des moyens d'obturation de l'ouverture de la poche, tels qu'une fermeture Éclair.

Au moins un des éléments textile peut être formé à partir d'une bande présentant deux bords libres opposés ramenés l'un en regard de l'autre sans qu'ils ne se chevauchent, et cousus l'un avec l'autre au moyen d'une surpiqure.
la bande peut avoir une forme de parallélogramme.
le manchon élastique peut présenter au repos une forme annulaire s'étendant autour d'un axe, et comprendre au moins un fil élastique de compression agencé de manière à suivre une trajectoire sensiblement hélicoïdale autour de et le long de l'axe.

Selon un autre aspect de l'invention, il est également proposé un vêtement ou une partie de vêtement, telle qu'une manche ou une jambe de pantalon, comprenant au moins un garrot conforme à la description qui précède.

### DESCRIPTION DES FIGURES

D'autres caractéristiques, buts et avantages de l'invention ressortiront de la description qui suit, qui est purement illustrative et non limitative, et qui doit être lue en regard des dessins annexés sur lesquels :
- La figure 1 est une vue en perspective de côté d'un support faisant partie d'un garrot, selon un mode de réalisation.
- Les figures 2 à 5 sont des vues montrant vue de dessus ou en perspective de côté deux éléments textiles faisant également partie d'un garrot, selon un mode de réalisation.
- La figure 6 est une vue en perspective de côté d'un manchon élastique faisant partie d'un garrot, selon un mode de réalisation.
- Les figures 7 à 9 sont des vues de dessus et de côté d'un élément d'entraînement partie d'un garrot, selon un mode de réalisation.
- Les figures 10 et 11 sont des vues de dessus et de dessous d'un élément de préhension faisant partie d'un garrot, selon un mode de réalisation.
- La figure 12 est une vue en perspective latérale d'un garrot selon un premier mode de réalisation, comprenant les éléments représentés sur les figures précédentes.
- La figure 13 est une vue en coupe partielle longitudinale du garrot représenté en figure 12.
- La figure 14 est une vue en coupe longitudinale du garrot représenté en figure 12, dans une configuration repliée.
- Les figures 15 et 16 sont deux vues de dessus d'un élément de support faisant partie d'un garrot selon un deuxième mode de réalisation.
Sur l'ensemble des figures, les éléments similaires portent des références identiques.

### DESCRIPTION DETAILLEE DE L'INVENTION

Est illustré sur les figures annexées un garrot 1 comprenant un support 2, un manchon élastique 4, un élément d'entraînement 6, et un élément de préhension 8 mobile par rapport au support 2.

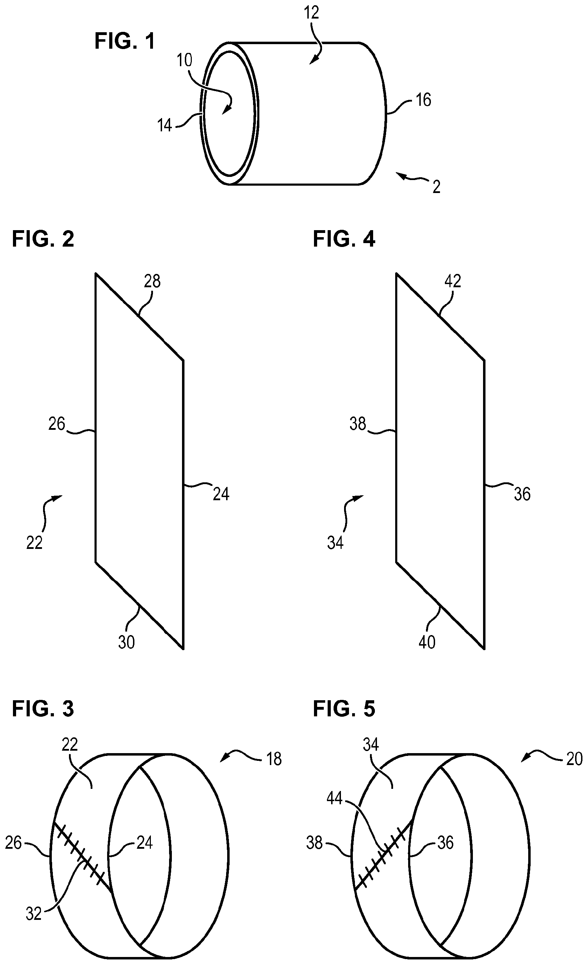

En référence à la **figure 1****,** le support 2 est propre à entourer un membre - membre supérieur (bras, avant-bras, main) ou membre inférieur (cuisse, jambe, pied) d'un porteur.

Le support 2 comprend ou est constitué d'un tube en matière plastique (tel que du polypropylène), ou en carbone. Le support est de manière générale réalisé dans un matériau empêchant une rétractation du manchon élastique 4, qui sera décrit plus loin.

Le support 2 peut présenter une forme cylindrique et/ou de révolution. Alternativement, le tube en matière plastique peut avoir une forme plus complexe, sensiblement complémentaire d'un membre nu ou d'un membre entouré par un vêtement.

Le support 2 présente une surface interne 10 destinée à être orientée vers un membre du porteur, et une surface externe 12 opposée à la surface interne, un premier bord 14 et un deuxième bord 16 opposé au premier bord 14, les deux bords 14, 16 reliant chacun la surface interne 10 à la surface externe 12.

Par exemple, le support rigide 2 a un diamètre intérieur de 118 millimètres (+/-1mm), et a une épaisseur radiale de 3 millimètres (+/- 0.2 millimètres) mesurée entre sa surface interne 10 et sa surface externe 12, et a une largeur, mesurée entre les deux bords 14 et 16, de 40 millimètres (+/- 0.1 millimètres).

En référence aux **figures 2** à **5****,** le garrot 1 comprend par ailleurs un élément textile interne 18 et un élément textile externe 20.

L'élément textile interne 18 est destiné à recouvrir la surface interne 10 du support rigide 2. De la sorte, le support rigide 2 ne peut pas entrer en contact direct avec la peau du membre. En particulier, lorsque le support rigide 2 est en matière plastique, cette matière plastique est susceptible de provoquer des allergies cutanées ; l'élément textile interne 18 permet dans ce cas d'éviter de telles allergies.

L'élément textile interne 18 est fabriqué à partir d'une première bande de matériau 22 ayant de préférence une forme de parallélogramme. Les deux grands côtés opposés 24, 26 de la première bande 22 font par exemple chacun 370 millimètres de long et les deux petits côtés opposés 28, 30 de la première bande 22 font par exemple chacun 37 millimètres de long. L'élément textile interne 18 présente par exemple une épaisseur de 1 à 2 millimètres.

Les deux petits côtés 28, 30 sont reliés l'un à l'autre sans se chevaucher, au moyen d'une surpiqure 32. Une telle liaison a pour avantage d'être particulièrement confortable pour le porteur du garrot 1, car ne crée pas de surépaisseur. De plus, la forme en parallélogramme de la bande 22 permet d'appliquer une surpiqure 32 de plus grande longueur que si la bande avait été de forme rectangulaire, ce qui rend cette liaison comparativement plus solide.

Ainsi refermé sur lui-même, l'élément textile interne 18 présente une forme annulaire, comme cela est visible sur la **figure 3****.**

L'élément textile interne 18 est idéalement réalisé en polyethersulfone (PES) déperlant non feu, ou en polyamide (PA) déperlant de type cordura 100% ou autre avec traitement non feu. Il présente par ailleurs un poids de 120 à 450 g/m². L'élément textile interne 18 comprend en outre de préférence une armure de type toile, de type sergé ou toute autre type d'armure.

Par ailleurs, l'élément textile externe 20 est destiné à recouvrir la surface externe 12 du support rigide 2.

L'élément textile externe 20 est fabriqué à partir d'une deuxième bande de matériau 34 de préférence de forme parallélépipédique. Les deux grands côtés 38, 40 opposés de la deuxième bande 34 font exemple chacun 370 millimètres de long et les deux petits côtés opposés 40, 42 de la deuxième bande 34 font par exemple chacun 35 millimètres de long. L'élément textile externe 20 présente une épaisseur de 1 à 2 millimètres.

Comme pour l'élément textile interne 18, les deux petits côtés 40, 42 de la deuxième bande 34 sont reliés l'un à l'autre sans se chevaucher, au moyen d'une surpiqure. Ceci a pour avantage d'éviter toute surépaisseur indésirable.

L'élément textile externe 20 est de préférence rigide (selon un sens de chaîne et un sens de trame). L'élément textile externe 20 peut être réalisé dans un matériau identique au matériau de l'élément textile interne 18. Alternativement, l'élément textile externe 20 peut être réalisé à base de polyuréthane et de polyethersulfones (PES) ou de polyamique (PA), ou en une gomme obtenue par vulcanisation d'hévéa. Toutes les autres propriétés énoncées ci-dessus pour l'élément textile interne 18 (poids, caractère déperlant, type d'armure) sont également applicables à l'élément textile externe 20.

Au moins un des éléments textiles 18, 20 peut présenter sur l'une de ses deux faces, des fils anti-glissements (non-illustrés sur les figures), cette face étant destinée à reposer contre l'une des surfaces interne 10 ou externe 12 du support 2, selon le cas. Grâce à ces fils anti-glissements, une rotation du support 2 relatif à l'élément textile comprenant de tels fils anti-glissement peut être empêchée. Une telle rotation est indésirable lorsque le support a une forme qui n'est pas de révolution, car serait susceptible de gêner le porteur du garrot 1.

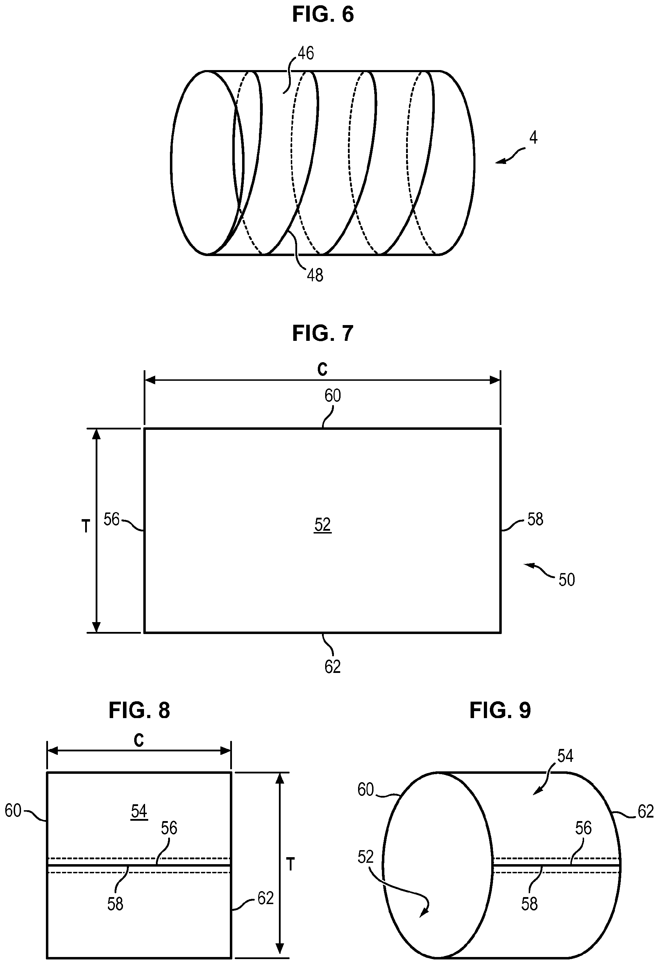

En référence à la **figure 6****,** le manchon élastique 4 est destiné à être positionné autour d'un membre dans un état étiré, et à se rétracter, par retour élastique, vers les vaisseaux sanguins de ce membre, de sorte à interrompre une hémorragie.

Le manchon élastique 4 est réalisé par exemple à partir d'une bande élastique refermée sur elle-même de sorte à prendre, au repos, une forme de tube. Le manchon élastique 4 est susceptible d'être étiré radialement, c'est-à-dire que son diamètre peut être augmenté.

Typiquement la bande élastique à partir de laquelle le manchon élastique 4 est formé fait, au repos, 20 centimètres de longueur sur 10 centimètres de largeur. Son poids est de préférence compris entre 15 à 25 grammes.

Le manchon élastique 4 comprend un tricot 46, par exemple constitué d'un fil d'habillage. Le fil d'habillage est par exemple en polyamide.

Le manchon élastique 4 comprend par ailleurs au moins un fil de compression élastique 48. Le fil de compression élastique 48 est entrelacé au tricot 46 de manière à suivre une trajectoire sensiblement hélicoïdale autour de l'axe du tube formé par le manchon élastique 4. Un avantage d'une telle forme de fil de compression élastique est que, même s'il est rompu alors que le manchon élastique 4 est dans un état étiré radialement, il peut tout de même se rétracter, par retour élastique, vers sa configuration rétractée, d'une manière relativement uniforme. En effet, la rupture du fil de compression donne naissance à deux ou plusieurs portions hélicoïdales disjointes, chaque portion demeurant rétractable. Le fil de compression élastique 48 comprend par exemple de 20 à 40 % d'élasthanne et de 80 à 60 % de polyamide.

Par ailleurs, l'élément d'entraînement 6 a pour fonction d'entraîner le manchon élastique 4, lorsque l'élément de préhension 8 est déplacé manuellement par rapport au support 2, depuis une position étirée autour du support 2 vers une position de garrotage dans laquelle le manchon élastique 4 entoure une deuxième partie du membre adjacente à la première partie du membre mais non entourée par le support 2, de sorte que le manchon élastique 4 puisse se rétracter par retour élastique, et ainsi comprimer la deuxième partie du membre.

En référence aux **figures 7 à 9**, l'élément d'entraînement 6 du garrot 1 est réalisé à partir d'une bande 50 appelée par la suite « bande d'entraînement » pour la distinguer des bandes à partir desquelles sont réalisés les éléments textiles interne 18 et externe 20.

La bande d'entraînement 50 comprend :
- une première surface 52, une deuxième surface 54 opposée à la première surface 52,
- un premier bord 56 et un deuxième bord 58 opposé au premier bord 56, les deux bords 56, 58 reliant la première surface 52 à la deuxième surface 54, et s'étendant chacun parallèlement à une direction T de trame de la bande 50,
- un troisième bord 60 et un quatrième bord 62 opposé au troisième bord, les deux bords reliant également la première surface à la deuxième surface, et s'étendant parallèlement à une direction de chaîne C de la bande perpendiculaire à la direction de trame T.

La bande d'entraînement 50, par exemple rectangulaire, présente une longueur, mesurée parallèlement à la direction de chaîne C, de 35 centimètres, et une largeur mesurée parallèlement à la direction de trame T, de 25 centimètres.

La bande d'entraînement 50 est idéalement réalisée dans un tissu sergé 2/1 Z, éventuellement en taffetas ou dans tout autre type de croisure de fil. De préférence, son poids est compris entre 90 et 380 g/m².

La bande d'entraînement 50 est extensible parallèlement à la direction de trame T (c'est-à-dire que le premier bord 56 et le deuxième bord 58 peuvent être éloignés l'un de l'autre), mais non-extensible dans le sens de la direction de chaîne C (c'est-à-dire que le troisième bord 60 et le quatrième bord 62 ne peuvent pas être éloignés l'un de l'autre).

De préférence, la bande d'entraînement 50 comprend 2 à 8 % d'élasthanne et de 98 à 92% de polyamide de type cordura déperlant avec traitement non-feu ou de polyester déperlant non-feu. Alternativement, la bande d'entraînement 50 est réalisée en fibres d'aramides, tel qu'en poly(p-phénylènetéréphtalamide) (PPD-T) (connu sous la dénomination commerciale Kevlar), matériau avantageux pour sa solidité, ou en fibres naturelles, tel qu'en lin.

Pour former l'élément d'entraînement 6, les bords 56, 58 de la bande d'entraînement 50 sont positionnés l'un en regard de l'autre sans se chevaucher, et sont reliés entre eux au moyen d'une surpiqure. L'élément d'entraînement 6 présente ainsi une forme annulaire ayant un diamètre d'environ 17 cm (+/- 0,5 cm), comme représenté en figure 9.

La direction de chaîne C est parallèle à un axe autour duquel s'étend l'élément d'entraînement 6 dans sa configuration annulaire. Ainsi, l'élément d'entraînement 6 n'est pas extensible parallèlement à cet axe.

Par convention, on appelle « configuration déployée » la configuration de l'élément d'entraînement 6 dans laquelle il a une telle forme annulaire ; l'élément d'entraînement 6 peut être déformé de sorte à adopter d'autres configurations. En effet, comme la bande d'entraînement 50 est extensible parallèlement au sens de trame T, l'élément d'entraînement 6 annulaire peut être ourlé sur lui-même une ou plusieurs fois autour de son axe, comme il sera vu dans la suite.

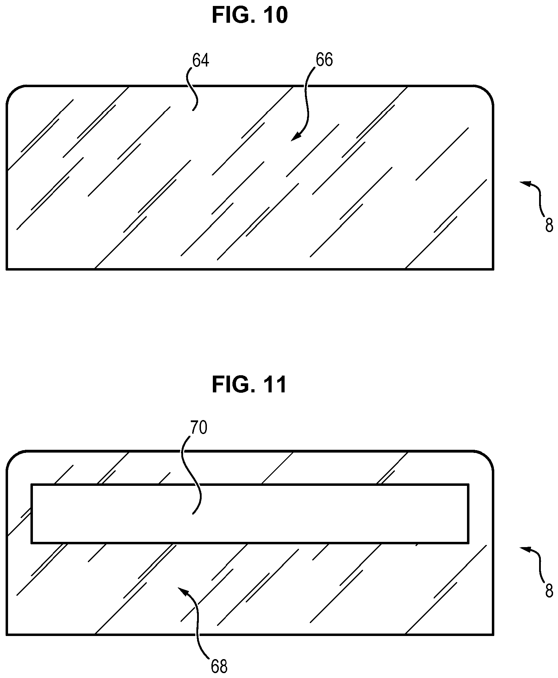

En référence aux **figures 10 et 11**, l'élément de préhension 8 comprend un rabat 64, par exemple sensiblement rectangulaire. Le rabat 64 présente deux surfaces opposées 66, 68 (l'une étant visible sur la **figure 10** et l'autre sur la figure 11).

Le rabat 64 présente par exemple une longueur de 11 cm et une largeur de 4 cm.

Le rabat 64 est de préférence réalisé dans un matériau plastique souple contrecollé textile, ou en un matériau textile laqué ou enduit double face. Très préférentiellement, le rabat 64 est un article non-feu déperlant en polyester à 100%.

L'élément de préhension 8 comprend sur l'un de ses deux faces, un élément de fixation 70 propre à être fixé de manière amovible à un vêtement.

Typiquement, l'élément de fixation 70 est une bande de velours ou à crochets destinée à former, avec un élément de fixation complémentaire fixé au vêtement, une liaison velours-crochet.

L'élément de fixation 70 est par exemple rectangulaire et fait par exemple 1 cm de largeur et 9 cm de longueur.

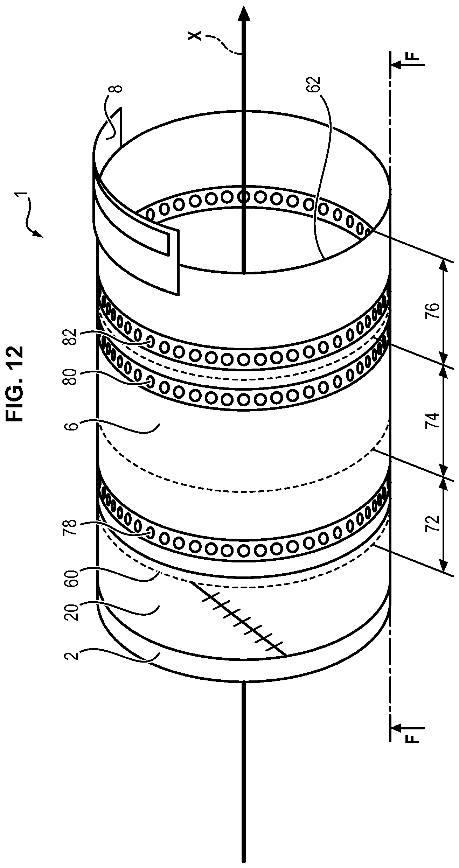

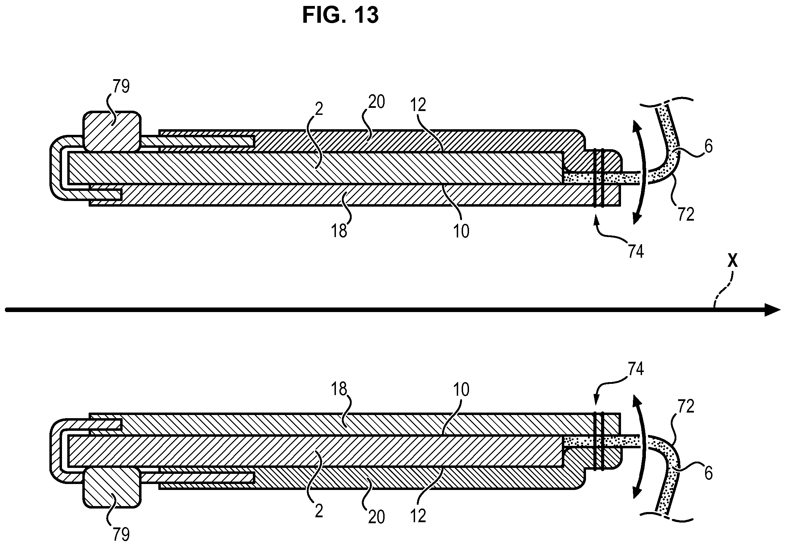

En référence aux **figures 12 à 14**, les deux éléments textiles 18 et 20, le support 2, le manchon élastique 4, l'élément d'entraînement 6 et l'élément de préhension 8 sont assemblés de la manière suivante pour former le garrot 1.

Les éléments textiles 18 et 20, le support 2, le manchon élastique 4 et l'élément d'entraînement 6 sont agencés de manière coaxiale, autour d'un axe X.

L'élément d'entraînement 6 comprend successivement, depuis le bord 60 jusqu'au bord 62 qui lui est opposé : une première portion 72, une deuxième portion 74, et une troisième portion 76. Sur la **figure 12**, les deux jonctions entre les portions 72 et 74, et entre les portions 74 et 76 sont représentées par deux lignes en pointillés. On verra dans la suite que ces portions ont des fonctions différentes.

La première portion 72 comprend une première pluralité d'orifices 78, la deuxième portion 74 comprend une deuxième pluralité d'orifices 80, et la troisième portion 76 comprend une troisième pluralité d'orifices 82.

Chaque orifice 78, 80, 82 débouche dans les deux surfaces opposées 52, 54 de l'élément d'entraînement 6.

Les orifices de chaque pluralité d'orifices 78, 80, 82 sont répartis autour de l'axe X de l'élément d'entraînement 6 lorsque celui est dans sa configuration déployée, dans laquelle il a une forme annulaire. Les trois pluralités d'orifices 78, 80, 82 s'étendent dans des plans respectifs parallèles, à distance les uns des autres le long de l'axe de l'élément d'entraînement 6. On verra dans la suite qu'un cordon 86 est destiné à être passé à travers les orifices 78, 80, 82. Pour éviter une déchirure de l'élément d'entraînement 6 causée par un frottement d'un tel cordon 86 contre le bord d'un des orifices, au moins un des orifices 78, 80, 82 (par exemple tous) est pourvu d'un œillet.

L'élément textile externe 20 est positionné autour de l'élément textile interne 18. Ils sont fixés l'un par rapport à l'autre au moyen de coutures 74. Une poche annulaire est ainsi formée entre les deux éléments textile 18, 20. De préférence, la première portion 72 de l'élément d'entraînement 6 est prise en sandwich entre les deux éléments textile 18 et 20 ; la première portion 72 et les deux éléments textiles, ainsi superposés, sont cousus ensemble à l'aide des coutures 74.

Le support 2 est inséré dans la poche formée entre les deux éléments textiles interne et externe. Le bord 60 de l'élément d'entraînement 6, confiné entre les deux éléments textiles 18 et 20, participe à définir la poche et se trouve par conséquent en regard du support 2 logé dans la poche.

De préférence, la poche annulaire présente une ouverture annulaire permettant le retrait du support logé dans la poche, ou bien l'insertion d'un support dans la poche lorsque celle-ci est vide. Il est ainsi possible de remplacer un support 2 usagé, endommagé ou de forme inadaptée par un autre support 2.

L'ouverture annulaire de la poche se trouve à l'opposé du bord 60 de l'élément d'entraînement 6, par rapport au support 2 logé dans la poche.

Le garrot 1 comprend des moyens d'obturation 79 pour obturer l'ouverture de la poche, telle qu'une fermeture Éclair. L'ouverture de la poche est obturée à l'aide des moyens d'obturation 79.

Il est à noter que l'élément textile externe 20 ne recouvre pas totalement l'élément textile interne 18. L'élément textile interne a une largeur, mesurée parallèlement à l'axe X, plus grande que la largeur de l'élément textile externe, si bien qu'une portion extrémale de l'élément textile interne n'est pas recouverte par l'élément textile externe 20. Les moyens d'obturation sont agencés en regard de cette portion extrémale non recouverte. Une fois inséré dans la poche, le support 2 se trouvant ainsi entre, d'une part, les moyens d'obturation et l'élément textile externe 20, et d'autre part l'élément textile interne 18.

Par ailleurs, l'élément de préhension 8 est fixé à l'élément d'entraînement 6 au niveau de son bord 62. Plus précisément, le rabat 64 est cousu sur la surface 54 de l'élément d'entraînement 6, en sa troisième portion 76.

Ainsi, lorsque l'élément d'entraînement 6 est dans sa configuration déployée dans laquelle il a une forme annulaire, l'élément d'entraînement 6 s'étend entre le support 2 et l'élément de préhension 8. Par ailleurs, dans cette même configuration, la surface 54 est orientée radialement vers l'extérieur, et la surface opposée 52 orientée vers l'intérieur de l'élément d'entraînement 6 de forme annulaire.

L'élément d'entraînement 6 subit ensuite les déformations suivantes, rendues possibles par son caractère extensible dans le sens de trame T.

L'élément d'entraînement 6 est ourlé sur lui-même à 180 degrés par déformation de la bande d'entraînement 50 radialement vers l'extérieur, de sorte que la première portion 72 soit rabattue autour du support 2 (donc contre l'élément textile externe 20 qui entoure le support 2).

Le manchon élastique 4 est positionné autour du support 2 dans un état étiré.

Plus précisément, le manchon élastique 4 est positionné autour de et contre la surface 52 de la première portion 72 de l'élément d'entraînement 6 rabattue autour du support 2.

Bien entendu, le manchon élastique 4 présente, au repos, un diamètre inférieur au diamètre externe de la première portion 72 de l'élément d'entraînement 6 rabattu autour du support 2, de sorte que le manchon soit dans un état étiré lorsqu'il est positionné autour de la première portion 72.

L'élément d'entraînement 6 est ensuite à nouveau ourlé sur-lui-même une deuxième fois au niveau de la jonction entre la première portion 72 et la deuxième portion 74.

Plus précisément, la deuxième portion 74 est rabattue à 180 degrés autour de la première portion 72, par déformation de la bande d'entraînement 50 radialement vers l'extérieur, de sorte que la deuxième portion 74 recouvre le manchon élastique 4 étiré autour de la première portion 72.

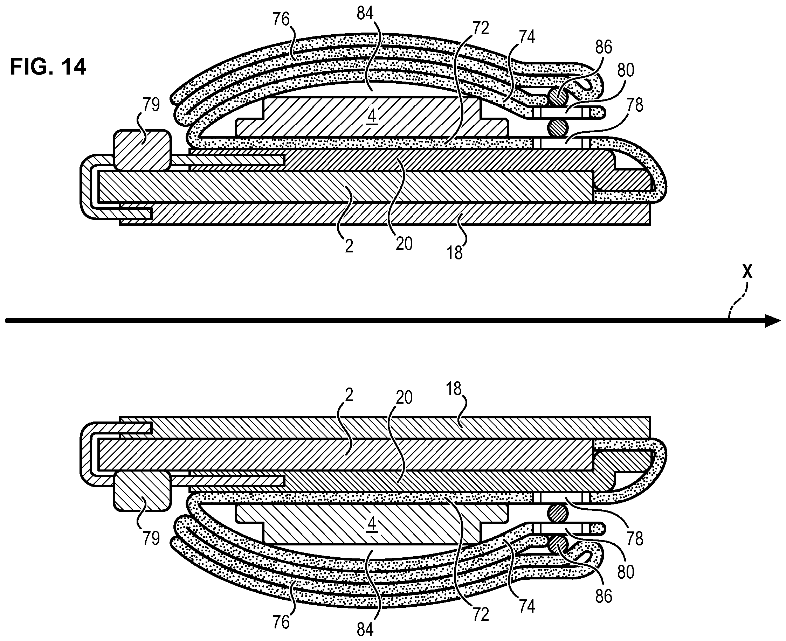

La première portion 72 et la deuxième portion 74 définissent ensemble une cavité 84, visible sur la figure 14, présentant un profil sensiblement en forme de U dont le fond correspond à la jonction entre la première portion 72 et la deuxième portion 74 de l'élément d'entraînement 6. Dans une telle configuration, le manchon élastique 4 est ainsi protégé contre des agressions extérieures au garrot 1 (causées par exemple par des projectiles ou des intempéries).

La deuxième portion 74 est rabattue autour de la première portion 72 de sorte qu'au moins un orifice 80 formé dans la deuxième portion 74 se trouve en regard d'un orifice 78 formé dans la première portion 72.

Au moins une paire d'orifices 78, 80 est agencée de sorte que le manchon élastique 4 logé dans la cavité 84 se trouve entre les deux orifices et le fond de la cavité 84 formé par la jonction entre la première portion 72 et la deuxième portion 74 de l'élément d'entraînement 6.

L'élément d'entraînement 6 est ensuite à nouveau ourlé sur-lui-même une troisième fois, au niveau de la jonction entre la deuxième portion 74 et la troisième portion 76.

Plus précisément, la troisième portion 76 est rabattue à 180 degrés radialement vers l'extérieur autour de la deuxième portion 74, par déformation de la bande d'entraînement 50, de sorte à former une couche supplémentaire de protection du manchon élastique 4 logé dans la cavité 84.

La troisième portion 76 est rabattue autour de la deuxième portion 74 de sorte qu'au moins un orifice 82 formé dans la troisième portion 76 se trouve en regard d'un orifice 80 formé dans la deuxième portion 74.

Très préférentiellement, la troisième portion 76 est rabattue autour de la deuxième portion 74 de sorte qu'au moins un orifice 82 formé dans la troisième portion 76, au moins un orifice 80 formé dans la deuxième portion 74, et un orifice 78 formé dans la première portion 72 soient tous les trois alignés.

Le garrot 1 comprend par ailleurs un cordon 86 présentant une portion d'extrémité préhensible, par exemple comprenant une boucle dans laquelle un doigt peut être passé.

Le cordon 86 est passé à travers au moins un des orifices formés dans la première portion 72, à travers au moins un des orifices formés dans la deuxième portion 74, voire à travers au moins un des orifices formés dans la troisième portion 76. Il est par ailleurs fait en sorte que l'extrémité préhensible du cordon 86 soit accessible depuis l'extérieur du garrot 1, de sorte à pouvoir être saisi et tiré par un utilisateur.

Les orifices 78, 80 et le cordon 86 forment ainsi des moyens de verrouillage de la deuxième portion 74 de l'élément d'entraînement 6 dans sa configuration rabattue sur la première portion 72 de l'élément d'entraînement 6.

Les orifices 80, 82 et le cordon 86 forment en outre des moyens de verrouillage de la troisième portion 76 de l'élément d'entraînement 6 dans sa configuration rabattue sur la deuxième portion 74 de l'élément d'entraînement 6. Il est à noter que la figure 14 n'est pas à la même échelle que la figure 12. La portion 76 représentée sur la figure 14 présente une longueur au moins égale à la longueur cumulée des portions 72 et 74.

Le cordon 86 est passé à travers au moins une paire d'orifices 78, 80 formés respectivement dans la première portion 72 et la deuxième portion 74 de sorte que le manchon élastique 4 logé dans la cavité 84 se trouve entre les deux orifices et le fond de la cavité 84. La portion du cordon 86 s'étendant depuis l'un de ces deux orifices 78, 80 jusqu'à l'autre orifice 78, 80 forme alors une butée empêchant le manchon élastique 4 logé dans la cavité 84 d'en sortir.

Ainsi, le cordon 86 a non seulement pour effet d'empêcher un déploiement de l'élément d'entraînement 6 vers sa configuration déployée représentée en figure 12, mais également pour effet d'empêcher une sortie inopinée du manchon élastique 4 hors de la cavité 84 même lorsque la deuxième portion 74 est rabattue autour de la première portion 72 de l'élément d'entraînement 6, comme illustré en figure 14.

L'assemblage des différentes pièces du garrot 1 est alors terminé. Le garrot 1 est à ce stade prêt à être fixé à un vêtement.

### Pré-installation du garrot 1 sur un vêtement

Le garrot 1 est positionné sur un vêtement, ou à une partie de vêtement, de telle sorte que le support 2 entoure une partie de vêtement destinée à envelopper un membre : cette partie est donc par exemple une manche ou bien une jambe de pantalon.

Dans une première variante, l'élément textile interne 18 repose contre une surface extérieure de cette partie de vêtement, voire est cousue avec elle. Dans une deuxième variante, l'élément textile interne 18 du garrot 1 fait partie elle-même de la partie de vêtement.

La partie de vêtement comprend trois portions successives : une portion proximale (dans le cas d'une manche, destinée à être proche d'une épaule), une portion intermédiaire prolongeant la portion proximale, et une portion distale (dans le cas d'une manche, destinée à être plus proche d'une main) prolongeant la portion intermédiaire.

Le garrot 1 est positionné de sorte que le support 2 s'étende autour de la portion intermédiaire de la partie de vêtement. La portion proximale et la portion distale de la partie de vêtement ne sont pas recouvertes par le support 2, mais sont situés de part et d'autre de celui-ci.

La manche comprend, sur la surface externe de sa portion proximale, un deuxième élément de fixation adapté pour coopérer avec l'élément de fixation 70 de l'élément de préhension 8 du garrot 1. Le deuxième élément de fixation est par exemple une bande à crochets lorsque l'élément de fixation 70 est une bande de velours, ou vice-versa.

L'élément de préhension 8 du garrot 1 surplombe la surface extérieure de la portion proximale de la partie de vêtement. Les deux éléments de fixation sont positionnés l'un contre l'autre, formant ensemble par exemple une liaison de type velours-crochet.

Un utilisateur peut alors revêtir le vêtement comprenant la partie de vêtement autour de laquelle le garrot 1 a ainsi été pré-positionné.

Par exemple, lorsque la partie de vêtement est une manche, et une fois que l'utilisateur a revêtu le vêtement comprenant une telle manche, la manche entoure un bras de cet utilisateur. Par exemple, la partie proximale de la manche entoure un tronçon proximal d'un bras, la partie intermédiaire entoure un tronçon intermédiaire du bras, et la partie distale entour une partie distale du bras.

Bien entendu, plusieurs garrots peuvent être pré-positionnés sur un même vêtement, par exemple quatre (deux pour les bras et deux pour les jambes).

### Utilisation du garrot par un porteur du garrot

Supposons à présent que le porteur du garrot subisse une blessure telle qu'il faille comprimer par garrottage les vaisseaux sanguins du tronçon distal de son bras. La zone à compresser est donc la surface extérieure du tronçon distal du bras. Le porteur porte un garrot 1 sur la manche entourant le bras blessé.

Avec son autre bras, valide, le porteur se saisit du rabat 64 de l'élément de préhension 8 et l'éloigne de son bras blessé de sorte que l'élément de fixation 70 et le deuxième élément de fixation se détachent l'un de l'autre.

A ce stade, il n'est pas encore possible de déplacer vers la zone à garrotter le manchon élastique 4 par rapport au support 2 autour duquel il s'étend dans un état étiré, à cause des moyens de verrouillage formés par le cordon 86 passés à travers les orifices 78, 80, 82.

Aussi, le porteur se saisit de la portion d'extrémité libre du cordon 86, accessible depuis l'extérieur du garrot 1. En tirant sur ce cordon 86, le cordon 86 se dévide hors des orifices 78, 80, 82, ce qui a pour effet non seulement d'ouvrir la cavité 84 dans laquelle est logée le manchon élastique 4, définie entre la première portion 72 et la deuxième portion 74 de l'élément d'entraînement 6, mais également d'autoriser un déplacement de la première portion 72 et de la deuxième portion 74 le long du membre, vers la zone à garrotter.

Le porteur replie à 180 degrés le rabat 64 vers la zone à garrotter, et tire le rabat 64 ainsi replié vers la zone à garrotter. Le rabat 64 étant fixé à l'élément d'entraînement 6, cette traction a pour effet d'entraîner la troisième portion 76 vers la zone à garrotter. Au cours de ce déplacement, la troisième portion 76 bascule de sa configuration rabattue autour de la deuxième portion 74 à une configuration déployée dans laquelle elle surplombe la zone à garrotter.

En continuant ce même mouvement de traction, la première portion 72 de l'élément d'entraînement 6 bascule à 180 degrés depuis sa configuration rabattue vers sa configuration déployée, et entraîne, par effet de levier, le manchon élastique 4 reposant sur la première portion 72 vers une position de garrottage, surplombant la zone à garrotter. Cet effet de levier est avantageux car le manchon élastique peut être déplacé moyennant une force de traction relativement faible. De la sorte, un utilisateur est à même d'exercer cette traction même lorsqu'il est blessé.

De plus, en raison du fait que la première portion 72 de l'élément d'entraînement 6 s'étende autour du support 2, l'effet de levier est appliqué par la première portion 72 de l'élément d'entraînement 6 sur tout le pourtour du manchon élastique 4 étiré qui l'entoure. De la sorte, les risques de rupture du manchon élastique 4 au cours du basculement de la première portion 72 depuis sa position rabattue vers sa position déployée sont réduits.

Par ailleurs, le fait que la première portion 72, et plus généralement l'élément d'entraînement 6, soit non-extensible parallèlement à l'axe X lui permet de mettre en œ uvre une transmission de mouvement plus efficace de l'élément de préhension 8 tracté au manchon élastique 4.

Une fois que la première portion 72 s'est déployée, le manchon élastique 4 se retrouve dans sa position de garrottage, autour de la zone à garrotter. La première portion 72 entoure alors le manchon élastique 4 entre la première portion 72 et la zone à garrotter.

Comme le manchon élastique 4 n'est plus maintenu, dans sa position de garrottage, dans un état étiré par le support 2, le manchon élastique 4 se rétracte librement par retour élastique, et comprime ainsi la zone à garrotter qu'il entoure.

L'invention ne se limite pas au mode de réalisation décrit précédemment, mais peut faire l'objet de nombreuses autres variantes.

Par exemple, dans le mode de réalisation précédemment décrit, il a été considéré que l'élément textile interne 18 fasse partie du vêtement ou cousu à celui-ci, et que l'élément de fixation du rabat 64 coopère avec un élément de fixation faisant partie du vêtement, par exemple cousu à celui-ci.

Dans un autre mode de réalisation, le garrot 1 comprend un élément additionnel de support 88 pour supporter le deuxième élément de fixation qui coopère avec l'élément de fixation 70 de l'élément de préhension 8.

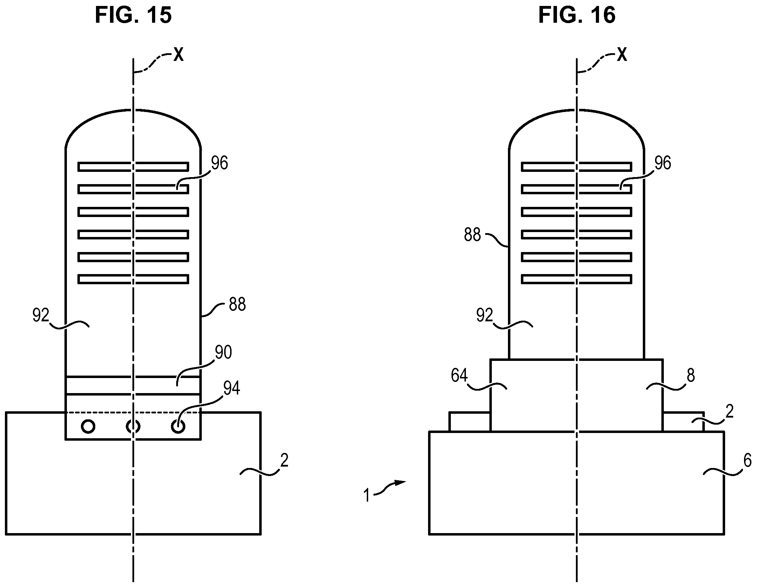

En référence à la **figure 15**, l'élément de support 88 se présente sous la forme d'une bande de support destinée à s'étendre le long d'une manche. Sur cette figure, le deuxième élément de fixation est référencé 90. Ce deuxième élément de fixation 90 est fixé sur une surface externe de l'élément de support 88.

L'élément de support 88 est fixé au support 2, par exemple via des rivets 94.

L'élément de support 88 présente par ailleurs une surface interne destinée à être orientée vers la manche, et une surface externe opposée à la surface interne, sur laquelle est agencé l'élément de fixation.

L'élément de support présente par ailleurs une pluralité de fentes parallèles 96. Les fentes 96 s'étendent transversalement à l'axe X autour duquel s'étend le support 2. Lorsque le garrot 1 est monté sur un bras, une épaulette de vêtement peut être passée à travers l'une des fentes 96 et refermée sur elle-même. Ainsi, la bande de support s'étend sur la portion proximale du bras, entre l'épaulette et le support 2.

En référence à la **figure 16**, le rabat 64 de l'élément de préhension 8 surplombe l'élément de support 88 et l'autre élément de fixation agencé sur sa surface extérieure. Ainsi, plutôt que de fixer directement le rabat 64 au vêtement, le rabat 64 est fixé à l'élément de support additionnel, ce qui est avantageux lorsqu'il n'est pas possible coudre d'élément de fixation au vêtement.

Par ailleurs, le fait de prévoir plusieurs fentes 96 parallèles permet d'adapter la pose de la bande de support à plusieurs tailles d'avant-bras.

## Revendications

1. Garrot (1) comprenant :
• un support (2) propre à entourer une première partie d'un membre,
• un manchon élastique (4) propre à être maintenu dans une position étirée autour du support (2), le support (2) empêchant une rétraction du manchon élastique (4),
• un élément de préhension (8) mobile par rapport au support (2),
• un élément d'entraînement (6) propre à entraîner le manchon élastique (4), lorsque l'élément de préhension (8) est déplacé manuellement par rapport au support (2), depuis sa position étirée autour du support vers une position de garrottage dans laquelle le manchon élastique (4) entoure une deuxième partie du membre, adjacente à la première partie du membre mais non entourée par le support (2), de sorte que le manchon élastique (4) puisse se rétracter par retour élastique, et ainsi comprimer la deuxième partie du membre,
**caractérisé en ce que** l'élément d'entraînement (6) comprend une première portion (72) et est propre à adopter:
• une configuration rabattue dans laquelle la première portion (72) s'étend entre le support (2) et l'ensemble du manchon élastique (4) maintenu dans la position étirée autour du support (2),
• une configuration déployée dans laquelle le manchon élastique (4), dans la position de garrottage, s'étend entre la première portion (72) et la deuxième partie du membre lorsque le support (2) entoure la première partie du membre,
et **en ce que** l'élément de préhension (8) est agencé par rapport à la première portion (72) pour qu'un déplacement de l'élément de préhension (8) par rapport au support (2) fasse basculer la première portion (72) de l'élément d'entraînement (6) depuis la configuration rabattue vers la configuration déployée, de sorte à déplacer, par effet de levier, le manchon élastique (4) depuis la position étirée vers la position de garrottage.

2. Garrot (1) selon la revendication précédente, dans lequel la première portion (72) est propre à :
• dans la configuration rabattue, entourer le support (2) de sorte que le manchon élastique (4) maintenu dans la position étirée s'étende autour de la première portion (72),
• dans la configuration déployée, entourer le manchon élastique (4).

3. Garrot (1) selon l'une des revendications 1 et 2, dans lequel la première portion (72) est propre à prendre une forme annulaire s'étendant autour d'un axe (X) dans la configuration déployée, et est réalisé dans un matériau non-extensible parallèlement à l'axe (X).

4. Garrot (1) selon l'une des revendications 1 à 3, dans lequel l'élément d'entraînement (6) comprend une deuxième portion (74) prolongeant la première portion (72), et dans lequel, dans la configuration rabattue, la deuxième portion (74) est rabattue sur la première portion (72) de sorte à former, entre les première et deuxième portion (74) de l'élément d'entraînement (6), une cavité (84) pour loger le manchon élastique (4) maintenu dans la position étirée.

5. Garrot (1) selon la revendication précédente, comprenant par ailleurs des moyens de verrouillage (78, 80, 86) de la deuxième portion (74) de l'élément d'entraînement (6) dans la configuration rabattue sur la première portion (72) de l'élément d'entraînement (6).

6. Garrot (1) selon la revendication précédente, dans lequel les moyens de verrouillage comprennent :
• au moins un orifice (78) formé dans la première portion (72),
• au moins un orifice (80) formé dans la deuxième portion (74),
• un cordon (86) propre à être passé à travers les deux orifices (78, 80), le cordon (86) présentant une portion d'extrémité libre susceptible d'être accessible depuis l'extérieur du garrot (1) et d'être tirée manuellement pour retirer le cordon (86) hors des orifices (78, 80), de sorte à déverrouiller les deux portions (72, 74) l'une par rapport à l'autre.

7. Garrot (1) selon la revendication précédente, dans lequel les orifices (78, 80) sont disposés l'un par rapport à l'autre pour qu'une portion du cordon (86) s'étendant depuis l'un des orifices (78) jusqu'à l'autre orifice (80) forme une butée empêchant le manchon élastique (4) logé dans la cavité (84) de sortir de la cavité (84).

8. Garrot (1) selon l'une des revendications 4 à 7, dans lequel l'élément d'entraînement (6) comprend une troisième portion (76) qui prolonge la deuxième portion (74) et qui est par ailleurs fixée à l'élément de préhension (8), la deuxième portion (74) s'étendant entre le manchon élastique (4) et la troisième portion (76) dans la configuration rabattue.

9. Garrot (1) selon l'une des revendications précédentes, dans lequel le déplacement de l'élément de préhension (8) suscitant l'entraînement, par l'élément d'entraînement (6), du manchon élastique (4) depuis la position étirée autour du manchon élastique (4) vers la position de garrottage est un déplacement causé par une traction manuelle exercée sur l'élément préhension le long du membre.

10. Garrot (1) selon l'une des revendications précédentes, dans lequel
• l'élément de préhension (8) comprend un élément de fixation (70) propre à fixer l'élément de préhension (8) de manière amovible à un vêtement pendant que le manchon élastique (4) est maintenu autour du support (2) dans sa position étirée, et
• l'élément de préhension (8) est fixé à l'élément d'entraînement (6) pour empêcher, lorsque l'élément de préhension (8) est fixé au vêtement, l'entraînement, par l'élément d'entraînement (6), du manchon élastique (4) depuis sa position étirée autour du support (2) vers sa position de garrottage.

11. Garrot (1) selon la revendication précédente, dans lequel l'élément de fixation (70) est propre à fixer l'élément de préhension (8) sur une portion de vêtement entourant une troisième partie du membre lorsque le support (2) entoure la première partie du membre, les deuxièmes et troisième parties du membre étant situées de part et d'autre de la première partie du membre.

12. Garrot (1) selon l'une des revendications 10 à 11, dans lequel l'élément de préhension (8) comprend un rabat (64) mobile par rapport à l'élément d'entraînement (6) entre :
• une configuration dépliée dans laquelle l'élément de fixation (70) est en regard du vêtement, de sorte à pouvoir être fixé au vêtement,
• une configuration repliée sur l'élément d'entraînement (6) en direction de la position de garrottage.

13. Garrot (1) selon l'une des revendications précédentes, dans lequel le support (2) présente une surface interne (10) propre à être orientée vers la première partie du membre, et une surface externe (12) opposée à la surface interne (10), le garrot (1) comprenant par ailleurs :
• un élément textile interne (18) recouvrant la surface interne (10), et/ou
• un élément textile externe (20) recouvrant la surface externe (12),
et dans lequel les deux éléments textiles (18, 80) définissent ensemble une poche pour contenir le support (2), la poche étant pourvue d'une ouverture par laquelle le support (2) est susceptible d'être retiré de la poche.

14. Garrot (1) selon l'une des revendications précédentes, dans lequel le manchon élastique (4) présente au repos une forme annulaire s'étendant autour d'un axe (X), et comprend au moins un fil élastique de compression agencé de manière à suivre une trajectoire sensiblement hélicoïdale autour de et le long de l'axe (X).

15. Vêtement ou partie de vêtement, telle qu'une manche ou une jambe de pantalon, comprenant au moins un garrot (1) selon l'une des revendications précédentes.

## Patentansprüche

1. Abbinder (1), umfassend:
• eine Unterlage (2), die imstande ist, einen ersten Teil einer Gliedmaße zu umgeben,
• eine elastische Manschette (4), die imstande ist, in einer gestreckten Position um die Unterlage (2) gehalten zu werden, wobei die Unterlage (2) ein Zusammenziehen der elastischen Manschette (4) verhindert,
• ein in Bezug auf die Unterlage (2) bewegliches Greifelement (8),
• ein Antriebselement (6), das imstande ist, die elastische Manschette (4), wenn das Greifelement (8) manuell in Bezug auf die Unterlage (2) verlagert wird, aus ihrer gestreckten Position um die Unterlage in eine Abbindeposition anzutreiben, in der die elastische Manschette (4) einen zweiten, zum ersten Teil der Gliedmaße benachbarten, aber nicht von der Unterlage (2) umgebenen Teil der Gliedmaße derart umgibt, dass sich die Manschette (4) durch elastischen Rückzug zusammenziehen und somit den zweiten Teil der Gliedmaße komprimieren kann,
**dadurch gekennzeichnet, dass** das Antriebselement (6) einen ersten Abschnitt (72) umfasst und imstande ist, anzunehmen:
• eine umgeschlagene Konfiguration, in welcher sich der erste Abschnitt (72) zwischen der Unterlage (2) und der gesamten elastischen Manschette (4) erstreckt, die in der gestreckten Position um die Unterlage (2) gehalten wird,
• eine ausgebreitete Konfiguration, in der sich die elastische Manschette (4) in der Abbindeposition zwischen dem ersten Abschnitt (72) und dem zweiten Teil der Gliedmaße erstreckt, wenn die Unterlage (2) den ersten Teil der Gliedmaße umgibt,
und dass das Greifelement (8) in Bezug auf den ersten Abschnitt (72) eingerichtet ist, damit eine Verlagerung des Greifelement (8) in Bezug auf die Unterlage (2) bewirkt, dass der erste Abschnitt (72) des Antriebselements (6) aus der umgeschlagenen Konfiguration in die ausgebreitete Konfiguration schwenkt, so dass die elastische Manschette (4) durch Hebelwirkung aus der gestreckten Position in die Abbindeposition verlagert wird.

2. Abbinder (1) nach vorangehendem Anspruch, wobei der erste Abschnitt (72) imstand ist:
• in der umgeschlagenen Konfiguration die Unterlage (2) derart zu umgeben, dass sich die in der gestreckten Position gehaltene elastische Manschette (4) um den ersten Abschnitt (72) erstreckt,
• in der ausgebreiteten Konfiguration die elastische Manschette (4) zu umgeben.

3. Abbinder (1) nach einem der Ansprüche 1 und 2, wobei der erste Abschnitt (72) imstande ist, eine Ringform anzunehmen, die sich in der ausgebreiteten Konfiguration um eine Achse (X) erstreckt und aus einem parallel zur Achse (X) nicht dehnbaren Material hergestellt ist.

4. Abbinder (1) nach einem der Ansprüche 1 bis 3, wobei das Antriebselement (6) einen zweiten Abschnitt (74) umfasst, der den ersten Abschnitt (72) verlängert und wobei der zweite Abschnitt (74) in der umgeschlagenen Konfiguration auf dem ersten Abschnitt (72) derart umgeschlagen ist, dass zwischen dem ersten und zweiten Abschnitt (74) des Antriebselements (6) ein Hohlraum (84) gebildet wird, um die in der gestreckten Position gehaltene elastische Manschette (4) aufzunehmen.

5. Abbinder (1) nach vorangehendem Anspruch, umfassend ferner Verriegelungsmittel (78, 80, 86) des zweiten Abschnitts (74) des Antriebselements (6) in der umgeschlagenen Konfiguration auf dem ersten Abschnitt (72) des Antriebselements (6).

6. Abbinder (1) nach vorangehendem Anspruch, wobei die Verriegelungsmittel umfassen:
• mindestens einen Durchbruch (78), der im ersten Abschnitt (72) ausgebildet ist,
• mindestens einen Durchbruch (80), der im zweiten Abschnitt (74) ausgebildet ist,
• eine Schnur (86), die imstande ist, durch die zwei Durchbrüche (78, 80) geführt zu werden, wobei die Schnur (86) einen freien Endabschnitt aufweist, der von außerhalb des Abbinders (1) erreichbar ist und manuell ziehbar ist, um die Schnur (86) aus den Durchbrüchen (78, 80) zu ziehen, so dass die zwei Abschnitte (72, 74) in Bezug zueinander entriegelt werden.

7. Abbinder (1) nach vorangehendem Anspruch, wobei die Durchbrüche (78, 80) in Bezug auf zueinander angeordnet sind, damit sich ein Abschnitt der Schnur (86), der sich von einem der Durchbrüche (78) bis zum anderen Durchbruch (80) erstreckt, einen Anschlag bildet, der verhindert, dass die im Hohlraum (84) untergebrachte elastische Manschette (4) den Hohlraum (84) verlässt.

8. Abbinder (1) nach einem der Ansprüche 4 bis 7, wobei das Antriebselement (6) einen dritten Abschnitt (76) umfasst, der den zweiten Abschnitt (74) verlängert und der ferner am Greifelement (8) befestigt ist, wobei sich der zweite Abschnitt (74) zwischen der elastischen Manschette (4) und dem dritten Abschnitt (76) in der umgeschlagenen Konfiguration erstreckt.

9. Abbinder (1) nach einem der vorangehenden Ansprüche, wobei die Verlagerung des Greifelements (8), die den Antrieb, durch das Antriebselement (6), der elastischen Manschette (4) aus der gestreckten Position um die elastische Manschette (4) in die Abbindeposition bewirkt, eine Verlagerung ist, die durch einen manuellen Zug verursacht wird, der auf das Greifelement entlang der Gliedmaße ausgeübt wird.

10. Abbinder (1) nach einem der vorangehenden Ansprüche, wobei
• das Greifelement (8) ein Befestigungselement (70) umfasst, das imstande ist, das Greifelement (8) lösbar an einer Bekleidung zu befestigen, währenddessen die elastische Manschette (4) um die Unterlage (2) in ihrer gestreckten Position gehalten wird, und
• das Greifelement (8) am Antriebselement (6) befestigt ist, um, wenn das Greifelement (8) an der Bekleidung befestigt ist, den Antrieb, durch das Antriebselement (6), der elastischen Manschette (4) aus ihrer um die Unterlage (2) gestreckten Position in ihre Abbindeposition zu verhindern.

11. Abbinder (1) nach vorangehendem Anspruch, wobei das Befestigungselement (70) imstande ist, das Greifelement (8) auf einem Bekleidungsabschnitt zu befestigen, der einen dritten Teil der Gliedmaße umgibt, wenn die Unterlage (2) den ersten Teil der Gliedmaße umgibt, wobei sich der zweite und dritte Teil der Gliedmaße beiderseits des ersten Teils der Gliedmaße befinden.

12. Abbinder (1) nach einem der Ansprüche 10 bis 11, wobei das Greifelement (8) einen Umschlag (64) umfasst, der in Bezug auf das Antriebselement (6) beweglich ist zwischen:
• einer entfalteten Konfiguration, in welcher das Befestigungselement (70) der Bekleidung zugewandt ist, so dass es an der Bekleidung befestigbar ist,
• einer auf das Antriebselement (6) in Richtung der Abbindeposition gefalteten Konfiguration.

13. Abbinder (1) nach einem der vorangehenden Ansprüche, wobei die Unterlage (2) eine innere Fläche (10) aufweist, die imstande ist, zum ersten Teil der Gliedmaße ausgerichtet zu sein, und eine äußere Fläche (12) gegenüber der inneren Fläche (10), wobei der Abbinder (1) ferner umfasst:
• ein inneres Textilelement (18), das die innere Fläche (10) abdeckt, und/oder
• ein äußeres Textilelement (20), das die äußere Fläche (12) abdeckt,
und wobei die zwei Textilelemente (18, 80) gemeinsam eine Tasche definieren, um die Unterlage (2) zu enthalten, wobei die Tasche mit einer Öffnung versehen ist, durch die die Unterlage (2) aus der Tasche herausziehbar ist.

14. Abbinder (1) nach einem der vorangehenden Ansprüche, wobei die elastische Manschette (4) in Ruhe eine Ringform aufweist, die sich um eine Achse (X) erstreckt, und mindestens einen elastischen Kompressionsfaden umfasst, der derart eingerichtet ist, dass er um und entlang der Achse (X) einen etwa schraubenförmigen Weg verfolgt.

15. Bekleidung oder Bekleidungsteil wie ein Ärmel oder ein Hosenbein, umfassend mindestens einen Abbinder (1) nach einem der vorangehenden Ansprüche.

## Claims

1. A tourniquet (1) comprising:
• a support (2) capable of surrounding a first part of a limb,
• an elastic sleeve (4) capable of being held in a stretched position around the support (2), the support (2) preventing a contraction of the elastic sleeve (4),
• a grip element (8) movable with respect to the support (2),
• a drive element (6) capable of driving the elastic sleeve (4), when the grip element (8) is manually displaced with respect to the support (2), from its stretched position around the support to a constricting position in which the elastic sleeve (4) surrounds a second part of the limb, adjacent to the first part of the limb but not surrounded by the support (2), so that the elastic sleeve (4) can retract itself by elastic return, and thus compress the second part of the limb,
**characterized in that** the drive element (6) comprises a first portion (72) and is capable of adopting:
• a folded configuration wherein the first portion (72) extends between the support (2) and the elastic sleeve (4) in its entirety maintained in the stretched position around the support (2),
• a deployed configuration wherein the elastic sleeve (4), in the constricting position, extends between the first portion (72) and the second part of the limb when the support (2) surrounds the first part of the limb,
and **in that** the grip element (8) is arranged with respect to the first portion (72) so that a displacement of the grip element (8) with respect to the support (2) causes the first portion (72) of the drive element (6) to shift from the folded configuration to the deployed configuration so as to displace, by lever action, the elastic sleeve (4) from the stretched position to the constricting position.

2. The tourniquet (1) according to the preceding claim, wherein the first portion (72) is capable of:
• in the folded configuration, surrounding the support (2) so that the elastic sleeve (4) held in the stretched position extends around the first portion (72),
• in the deployed configuration, surrounding the elastic sleeve (4).

3. The tourniquet (1) according to one of claims 1 and 2, wherein the first portion (72) is capable of taking on an annular shape extending around an axis (X) in the deployed configuration, and is manufactured in a non-extendable material parallel to the axis (X).

4. The tourniquet (1) according to one of claims 1 to 3, wherein the drive element (6) comprises a second portion (74) continuing the first portion (72) and wherein, in the folded configuration, the second portion (74) is folded over the first portion (72) so as to form, between the first and second portion (74) of the drive element (6), a cavity (84) for housing the elastic sleeve (4) held in the stretched position.

5. The tourniquet (1) according to the preceding claim, also comprising means (78, 80, 86) for locking the second portion (74) of the drive element (6) in the folded position on the first portion (72) of the drive element (6).

6. The tourniquet (1) according to the preceding claim, wherein the locking means comprise:
• at least one opening (78) formed in the first portion (72),
• at least one opening (80) formed in the second portion (74),
• a cord (86) capable of being passed through the two openings (78, 80), the cord (86) having a free end portion capable of being accessible from the exterior of the tourniquet (1) and of being pulled manually to withdraw the cord (86) out of the openings (78, 80), so as to unlock the two portions (72, 74) from one another.

7. The tourniquet (1) according to the preceding claim, wherein the openings (78, 80) are disposed with respect to one another so that a portion of the cord (86) extending from one of the openings (78) to the other opening (80) forms an abutment preventing the elastic sleeve (4) housed in the cavity (84) from leaving the cavity (84).

8. The tourniquet (1) according to one of claims 4 to 7, wherein the drive element (6) comprises a third portion (76) which continues the second portion (74) and which is also fixed to the grip element (8), the second portion (74) extending between the elastic sleeve (4) and the third portion (76) in the folded configuration.

9. The tourniquet (1) according to one of the preceding claims, wherein the displacement of the grip element (8) inducing the driving, by the drive element (6) of the elastic sleeve (4) from its stretched position around the elastic sleeve (4) to the constricting position, is a displacement caused by a manual pull exerted on the grip element along the limb.

10. The tourniquet (1) according to one of the preceding claim, wherein
• the grip element (8) comprises a fastening element (70) capable of fastening the grip element (8) removably to a piece of clothing while the elastic sleeve (4) is held around the support (2) in its stretched position, and
• the grip element (8) is fastened to the drive element (6) to prevent, when the grip element (8) is fastened to the piece of clothing, the driving, by the drive element (6), of the elastic sleeve (4) from its stretched position around the support (2) to its constricting position.

11. The tourniquet (1) according the preceding claim, wherein the fastening element (70) is capable of fastening the grip element (8) to a portion of a piece of clothing surrounding a third part of the limb when the support (2) surrounds the first part of the limb, the second and third parts of the limb being situated on either side of the first part of the limb.

12. The tourniquet (1) according to one of claims 10 to 11, wherein the grip element (8) comprises a flap (64) movable with respect to the drive element (6) between:
• an unfolded configuration wherein the fastening element (70) faces the piece of clothing, so as to be able to be fastened to the piece of clothing,
• a configuration folded back over the drive element (6) in the direction of its constricting position.

13. The tourniquet (1) according to one of the preceding claims, wherein the support (2) has an internal surface (10) capable of being oriented toward the first part of the limb, and an external surface (12) opposite to the internal surface (10), the tourniquet (1) also comprising:
• an internal textile element (18) covering the internal surface (10), and/or
• an external textile element (20) covering the external surface (12),
and wherein the two textile elements (18, 80) together define a pocket for containing the support (2), the pocket being provided with an opening by which the support (2) is capable of being withdrawn from the pocket.

14. The tourniquet (1) according to one of the preceding claims, wherein the elastic sleeve (4) has, at rest, an annular shape extending around an axis (X), and comprises at least one elastic compression yarn arranged so as to follow a substantially helical trajectory around and along the axis (X).

15. A piece of clothing or part of a piece of clothing, such as a sleeve or a trouser leg, comprising at least one tourniquet (1) according to one of the preceding claims.
